(19) Europäisches Patentamt
European Patent Office
Office européen des brevets

(11) Veröffentlichungsnummer: **0 024 624**
**B1**

(12) **EUROPÄISCHE PATENTSCHRIFT**

(45) Veröffentlichungstag der Patentschrift: **04.07.84**

(21) Anmeldenummer: **80104747.3**

(22) Anmeldetag: **12.08.80**

(00) **Verbunden mit 82101333.1 (europäische Anmeldenummer) durch Entscheidung vom 23.03.83.**

(51) Int. Cl.³: **C 07 C 43/315,**
C 07 C 41/48,
C 07 D 317/22,
C 07 D 317/16,
C 07 C 47/55,
C 07 C 45/29,
C 07 C 45/59,
C 07 C 47/575

(54) **4-Fluor-3-phenoxy-benzaldehyd-acetale und Verfahren zu deren Herstellung sowie Zwischenprodukte hierfür.**

(30) Priorität: **22.08.79 DE 2933979**

(43) Veröffentlichungstag der Anmeldung: **11.03.81 Patentblatt 81/10**

(45) Bekanntmachung des Hinweises auf die Patenterteilung: **04.07.84 Patentblatt 84/27**

(84) Benannte Vertragsstaaten: **BE CH DE FR GB IT LI NL**

(56) Entgegenhaltungen: **FR - A - 2 245 630**

(73) Patentinhaber: **BAYER AG**
**Konzernverwaltung RP Patentabteilung**
**D-5090 Leverkusen 1 Bayerwerk (DE)**

(72) Erfinder: **Fuchs, Rainer, Dr.**
**Roeberstrasse 8**
**D-5600 Wuppertal 1 (DE)**
Erfinder: **Maurer, Fritz, Dr.**
**Roeberstrasse 8**
**D-5600 Wuppertal 1 (DE)**
Erfinder: **Priesnitz, Uwe, Dr.**
**Severinstrasse 58**
**D-5650 Solingen 1 (DE)**
Erfinder: **Riebel, Hans-Jochem, Dr.**
**In der Beek 92**
**D-5600 Wuppertal 1 (DE)**
Erfinder: **Klauke, Erich, Dr.**
**Eichendorffweg 8**
**D-5063 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft 4-Fluor-3-phenoxy-benzaldehydacetale, ein Verfahren zu deren Herstellung, den 3-Brom-4-fluor-benzaldehyd und Acetale hiervon.

Es ist bekannt, daß man 4-Fluor-3-phenoxy-benzaldehyd, ein Zwischenprodukt für pestizid wirksame Pyrethroide, erhält, wenn man 4-Fluor-3-phenoxy-benzylbromid mit Hexamethylentetramin umsetzt und das Produkt dieser Umsetzung mit Säuren erhitzt (vergleiche DE—OS 2 709 264). Die Ausbeute ist jedoch bei dieser Synthesemethode, wie auch bei der Herstellung der Ausgangsverbindung aus 4-Fluor-3-phenoxy-toluol und N-Brom-succinimid, unbefriedigend.

Gegenstand der vorliegenden Erfindung sind:

(1) 4-Fluor-3-phenoxy-benzaldehyd-acetale der Formel I

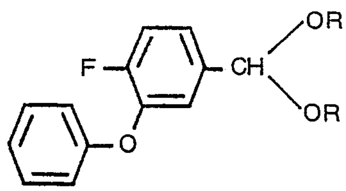

(I)

in welcher die beiden Reste

R einzeln für $C_{1-4}$-Alkyl oder zusammen für $C_{2-5}$-Alkandiyl (Alkylen) stehen;

(2) ein Verfahren zur Herstellung der Verbindungen der Formel (I), dadurch gekennzeichnet, daß man 3-Brom-4-fluor-benzaldehyd-acetale der Formel II

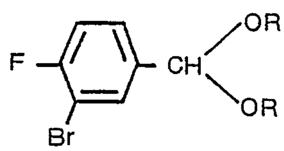

(II)

in welcher

R die oben angegebene Bedeutung hat,

mit Alkali- oder Erdalkali-phenolaten in Gegenwart von Kupfer oder Kupferverbindungen als Katalysatoren und in Gegenwart eines aprotisch polaren Verdünnungsmittels sowie gegebenenfalls in Gegenwart von Hilfsstoffen aus der Reihe der Alkali- oder Erdalkali-halogenide oder -carbonate und gegebenenfalls in Gegenwart eines basischen Entwässerungsmittels bei Temperaturen zwischen 100 und 200°C umsetzt;

(3) die Verwendung der Verbindungen der Formel (I) als Zwischenprodukte zur Herstellung von 4-Fluor-3-phenoxy-benzaldehyd durch Umsetzung mit Säuren nach an sich bekannten Acetalspaltungsmethoden; und

(4) 3-Brom-4-fluor-benzaldehyd sowie seine Acetale der Formel III und II

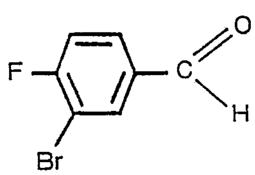

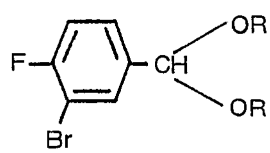

III II

in welcher die beiden Reste

R einzeln für $C_{1-4}$-Alkyl oder zusammen für C2-5-Alkandiyl (Alkylen) stehen.

(5) Verbindungen der Formel (II) werden erhalten, indem man 3-Brom-4-fluor-benzaldehyd der Formel III

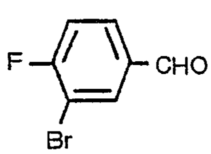

(III)

mit Alkan(di)olen gegebenenfalls in Gegenwart eines Katalysators und/oder eines Wasserbindemittels und gegebenenfalls unter Verwendung eines Verdünnungsmittels bei Temperaturen zwischen 0 und 150°C umsetzt.

(6) 3-Brom-4-fluor-benzaldehyd wird erhalten, indem man 3-Brom-4-fluor-benzole der Formel IV

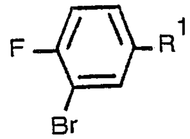

(IV)

in welcher

$R^1$ für $CH_3$ oder $—CH_2—OH$ steht

in üblicher Weise in den Aldehyd überführt.

Überraschenderweise kann über die vorstehend aufgeführten neuen Zwischenprodukte 4-Fluor-3-phenoxy-benzaldehyd auf einfachere Weise und in besserer Ausbeute als nach dem oben erwähnten bekannten Verfahren hergestellt werden.

In den 4-Fluor-3-phenoxy-benzaldehyd-acetalen der Formel (I) steht

R insbesondere für $—CH_2—CH_2—$.

Das unter (2) dargelegte Verfahren zur Herstellung der 4-Fluor-3-phenoxy-benzaldehyd-acetale ("Verfahren (2)") kann bei Verwendung von 3-Brom-4-fluor-benzaldehydpropylenacetal und Kaliumphenolat als Ausgangsverbindungen durch folgendes Reaktionsschema skizziert werden:

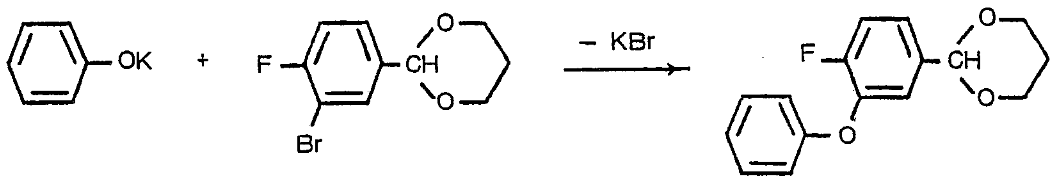


Alkali- oder Erdalkali-phenolate, welche bei Verfahren (2) als Ausgangsstoffe verwendet werden können, sind z.B. Natrium-, Kalium- und Magnesium-phenolat. Natriumphenolat wird als Ausgangsverbindung bevorzugt.

Als Katalysatoren werden Kupfer oder Kupferverbindungen verwendet. Als Beispiele hierfür seien Kupfer, Kupfer(I)oxid, Kupfer(II)oxid, Kupfer(I)chlorid und Kupfer(I)bromid genannt.

Als aprotisch polare Verdünnungsmittel werden bei Verfahren (2) vorzugsweise Dimethylformamid, Dimethylacetamid, N-Methylpyrrolidon, Dimethylsulfoxid, Tetramethylensulfon, Hexamethylphosphonsäuretriamid und Bis-(2-methoxyethyl)-ether (Diglyme) verwendet. Letzteres wird besonders bevorzugt.

Hilfsstoffe aus der Reihe der Alkali- oder Erdalkalihalogenide oder -carbonate sind z.B. Kalium- und Magnesiumcarbonat. Diese Hilfsstoffe werden vorzugsweise dann verwendet, wenn Natriumphenolat als Ausgangsverbindung eingesetzt wird.

Basische Entwässerungsmittel, welche bei Verfahren (2) verwendet werden können, sind z.B. Natriumhydroxid, Kaliumhydroxid, Calciumhydroxid, Kaliumcarbonat, Natriumamid, Natriumhydrid und Calciumhydrid.

Die Reaktionstemperatur wird bei Verfahren (2) zwischen 100 und 200°C, vorzugsweise zwischen 130 und 170°C gehalten. Das Verfahren wird gewöhnlich unter Normaldruck durchgeführt.

Auf 1 Mol 3-Brom-4-fluor-benzaldehyd-acetal der Formel (II) setzt man 1 bis 1,5 Mol, vorzugsweise 1 bis 1,2 Mol Phenolat, 0,01 bis 0,5 Mol, vorzugsweise 0,1 bis 0,5 Mol Kupferkatalysator, 100 bis 400 ml Verdünnungsmittel, gegebenenfalls 0,01 bis 0,5 Mol eines Hilfsstoffes aus der Reihe der Alkali- oder Erdalkali-halogenide oder -carbonate und gegebenenfalls bis zu 0,2 Mol eines Entwässerungsmittels ein.

In einer bevorzugten Ausführungsform von Verfahren (2) wird das Phenolat in einem Verdünnungsmittel, zu dem man gegebenenfalls ein Entwässerungsmittel gegeben hat, vorgelegt, mit dem Kupferkatalysator und gegebenenfalls mit dem Hilfsstoff aus der Reihe der Alkali- oder Erdalkalihalogenide oder -carbonate versetzt und das Gemisch wird auf die Reaktionstemperatur aufgeheizt. Dann wird das 3-Brom-4-fluor-benzaldehyd-acetal eindosiert und das Gemisch bis zum Reaktionsende gerührt. Zur Aufarbeitung, welche nach üblichen Methoden erfolgen kann, verdünnt man beispielsweise mit Toluol, filtriert und destilliert vom Filtrat das Lösungsmittel unter vermindertem Druck ab, wobei das Rohprodukt als Rückstand verbleibt.

Die Verbindungen der Formel (I) können zur Herstellung von 4-Fluor-3-phenoxybenzaldehyd, welcher als Zwischenprodukt für Pyrethroide bekannt ist (vergleiche DE—OS 2 709 264), verwendet werden. Die Herstellung von 4-Fluor-3-phenoxy-benzaldehyd durch Acetalspaltung von Verbindungen der Formel (I) mit Säuren kann bei Einsatz von 4-Fluor-3-phenoxy-benzaldehyd-propylenacetal durch folgendes Formelschema skizziert werden:

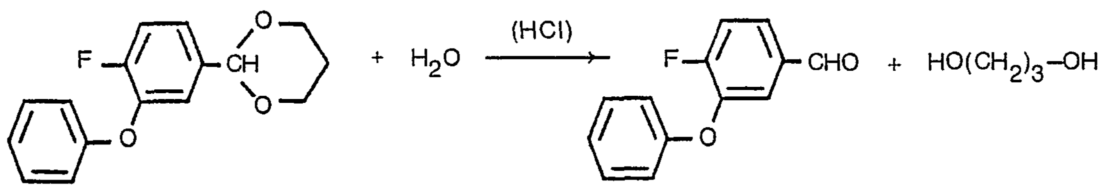


Die Acetalspaltung zur Herstellung von 4-Fluor-3-phenoxy-benzaldehyd kann nach üblichen Methoden durchgeführt werden. In einer bevorzugten Verfahrensweise werden die Verbindungen der Formel (I) in wässrigem Alkohol gelöst und mit der katalytischen Menge einer starken Säure, wie z.B. Salzsäure, bei Raumtemperatur stehen gelassen. Nach einigen Stunden wird das Reaktionsgemisch mit einem mit Wasser nicht mischbaren Lösungsmittel, wie z.B. Toluol, versetzt, die organische Phase abgetrennt, mit Wasser gewaschen, getrocknet, filtriert und destilliert.

In den als Zwischenprodukte zu verwendenden 3-Brom-4-fluor-benzaldehyd-acetalen der Formel (II) steht.

R insbesondere für $—CH_2—CH_2—$.

Das unter (5) dargelegte Verfahren zur Herstellung der 3-Brom-4-fluor-benzaldehyd-acetale ("Verfahren (5)") kann bei Verwendung von Propan-1,3-diol durch folgendes Reaktionsschema skizziert werden:

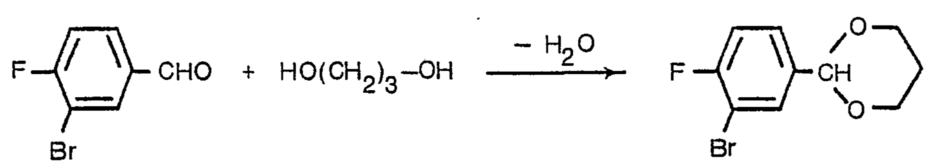


Alkanole bzw. Alkandiole, welche als Acetalisierungsmittel bei Verfahren (5) eingesetzt werden können, sind z.B. Methanol, Ethanol, Propanole und Butanole sowie Ethan-1,2-diol (Ethylenglycol) und Propan-1,3-diol. Ethylenglycol wird als Acetalisierungskomponente besonders bevorzugt.

Geeignete Katalysatoren sind z.B. Chlorwasserstoff, Bromwasserstoff, Schwefelsäure, p-Toluolsulfonsäure, Bortrifluorid, Zinkchlorid und saure Ionenaustauscherharze.

Geeignete Wasserbindemittel sind z.B. Orthoameisensäuretriethylester, Dimethylsulfit und Chlortrimethylsilan.

Verfahren (5) wird gegebenenfalls in Gegenwart von Verdünnungsmitteln durchgeführt. Als solche sind insbesondere Lösungsmittel geeignet, mit denen Wasser durch azeotrope Destillation aus dem Reaktionsgemisch entfernt werden kann. Als Beispiele seien Benzol, Toluol und Xylol genannt.

Die Reaktionstemperatur liegt bei Verfahren (5) zwischen 0 und 150°C, vorzugsweise zwischen 20 und 120°C. Das Verfahren wird im allgemeinen bei Normaldruck durchgeführt.

Auf 1 Mol 3-Brom-4-fluor-benzaldehyd setzt man 1 bis 1,5 Moläquivalente, vorzugsweise 1 bis 1,2 Moläquivalente Alkan(di)ol und gegebenenfalls 2 bis 3 Mol, vorzugsweise 2 bis 2,5 Mol eines Wasserbindemittels ein.

In einer bevorzugten Ausführungsform von Verfahren (5) werden 3-Brom-4-fluor-benzaldehyd, das Alkan(di)ol und ein Wasserbindemittel vermischt und einige Stunden erhitzt. Zur Aufarbeitung, welche nach üblichen Methoden erfolgen kann, wird beispielsweise mit einem mit Wasser nicht mischbaren Lösungsmittel, wie z.B. Toluol, verdünnt, die Lösung mit Eiswasser gewaschen, getrocknet, filtriert und destilliert.

Das Verfahren zur Herstellung von 3-Brom-4-fluor-benzaldehyd aus 3-Brom-4-fluor-benzylalkohol ("Verfahren (6)") wird vorzugsweise unter Verwendung von Verdünnungsmitteln durchgeführt. Als solche kommen gegebenenfalls Wasser und/oder aliphatische oder aromatische, gegebenenfalls halogenierte Kohlenwasserstoffe, wie z.B. Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol in Frage.

Die Reaktionstemperatur liegt bei Verfahren (6) zwischen 0 und 100°C, vorzugsweise zwischen 10 und 60°C. Das verfahren wird gewöhnlich bei Normaldruck durchgeführt.

In einer bevorzugten Variante (a) von Verfahren (6) wird als Oxidationsmittel Chrom(VI)oxid-Pyridin-Chlorwasserstoff (1/1/1) verwendet. Zu einer Lösung dieses Oxidationsmittels, beispielsweise in Methylenchlorid, gibt man einer Lösung von 3-Brom-4-fluor-benzyl-alkohol, ebenfalls in Methylenchlorid, und rührt das Gemisch bis zum Reaktionsende. Zur Aufarbeitung wird, gegebenenfalls nach Abdekantieren von ungelösten Komponenten, destilliert.

In einer zweiten bevorzugten Variante (b) von Verfahren (6) wird Salpetersäure als Oxidationsmittel und Wasser als Verdünnungsmittel verwendet. Hierzu gibt man den 3-Brom-4-fluor-benzylalkohol und rührt das Gemisch einige Stunden bei Raumtemperatur. Zur Aufarbeitung wird mit Natronlauge alkalisiert, mit einem mit Wasser nicht mischbaren Lösungsmittel, wie z.B. Toluol, extrahiert, oder

Extrakt mit Wasser gewaschen, getrocknet, filtriert und destilliert.

In einer dritten bevorzugten Variante (c) von Verfahren (6) wird Chromsäure bzw. Dichromat und Schwefelsäure als Oxidationsmittel verwendet. Verdünnungsmittel ist hierbei vorzugsweise ein Zweiphasensystem aus Wasser und einem der oben genannten organischen Lösungsmittel. Als Katalysatoren werden vorzugsweise Verbindungen verwendet, welche zum Transfer von Anionen aus Wasser in organische Lösungsmittel geeignet sind. Beispiele hierfür sind Benzyl-triethyl-ammonium-hydrogensulfat, Tetrabutyl-ammonium-bromid und Methyl-tricapryl-ammonium-chlorid (Aliquat 336).

Zur Durchführung der Verfahrensvariante (6 c) wird 3-Brom-4-fluor-benzylalkohol in einem organischen Lösungsmittel, wie z.B. Methylenchlorid, vorgelegt und dazu werden Schwefelsäure, Wasser, Katalysator und Dichromat gegeben. Das Reaktionsgemisch wird einige Stunden gerührt. Zur Aufarbeitung wird mit Wasser verdünnt, die organische Phase abgetrennt, mit Wasser gewaschen, getrocknet, filtriert und destilliert.

3-Brom-4-fluor-benzylalkohol erhält man, wenn man 3-Brom-4-fluor-benzoylfluorid mit einem Hydridkomplex, wie z.B. Natriumtetrahydridoborat, gegebenenfalls unter Verwendung eines Verdünnungsmittels, wie z.B. iso-Propanol, bei Temperaturen zwischen 0 und 50°C umsetzt. Das Reaktionsgemisch wird bis zum Ende der Umsetzung gerührt, mit Eiswasser verdünnt und angesäuert. Dann wird mit einem mit Wasser nicht mischbaren Lösungsmittel, wie z.B. mit Methylenchlorid, extrahiert, der Extrakt getrocknet, filtriert und destilliert.

Das als Ausgangsverbindung zu verwendende 3-Brom-4-fluor-benzoylfluorid ist Gegenstand der DE—OS 2 915 738.

Man erhält dieses Verbindung, wenn man 4-Chlor-benzoylchlorid durch Umsetzung mit Kaliumfluorid in 4-Fluor-benzoylfluorid umwandelt und letzteres zu 3-Brom-4-fluor-benzoylfluorid bromiert gemäß nachstehendem Formelschema:

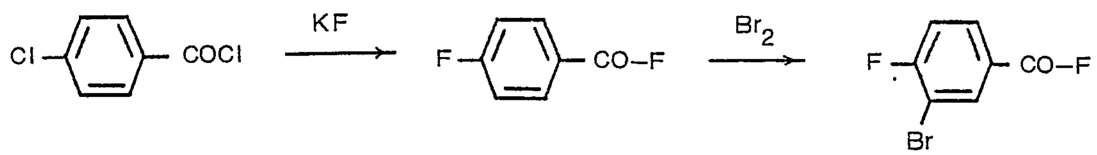


4-Chlor-benzoylchlorid wird mit Kaliumfluorid beispielsweise in Tetramethylensulfon bei Temperaturen zwischen 200 und 220°C umgesetzt und das Reaktionsgemisch destillativ aufgearbeitet. Man erhält 4-Fluor-benzoylfluorid vom Siedepunkt 53°C/20 mBar (Brechungsindex: $n_D^{20} = 1{,}4792$).

4-Fluor-benzoylfluorid wird mit elementarem Brom in Gegenwart von 1% Eisen(III)chlorid bei 70 bis 75°C umgesetzt. Bei einem Ansatz von 1 Mol erhält man nach Destillation 40 g unverändertes Ausgangsmaterial zurück und 182 g eines Gemisches von 3-Brom-4-fluor-benzoylfluorid (Siedepunkt: 82—83°C/15 mBar; Brechungsindex: $n_D^{20} = 1{,}5315$; Schmelzpunkt: 32—34°C) und 3-Brom-4-fluor-benzoylbromid (Siedepunkt: 123°C/15 mBar; Schmelzpunkt: 35—37°C).

Der nach Verfahren (6) herzustellende 3-Brom-4-fluor-benzaldehyd (III) kann prinzipiell auch nach anderen Methoden erhalten werden, beispielsweise durch Bromierung von 4-Fluor-benzaldehyd oder durch Seitenkettenhalogenierung von 3-Brom-4-fluor-toluol und anschließende Sommelet-Reaktion.

## Beispiel 1

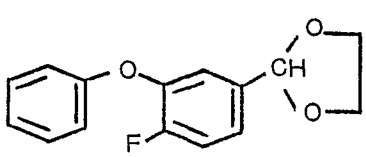

Zu einer Suspension von 3,2 g (27,5 mMol) Natriumphenolat in 3 ml Diglyme gibt man zur Entwässerung 0,1 g (3,5 mMol) Natriumhydrid und versetzt die Mischung dann mit 0,05 g Kupfer(I)oxid (0,35 mMol) sowie mit 0,5 g (6,5 mMol) Kaliumchlorid. Das Reaktionsgemisch wird unter Inertgas (z.B. Argon) auf 155°C erhitzt und bei dieser Temperatur mit 6,2 g (25 mMol) 3-Brom-4-fluor-benzaldehyd-ethylenacetal versetzt. Man rührt 7 Stunden bei 155°C nach, gibt nach Abkühlen auf Raumtemperatur 50 ml Toluol zu und saugt vom anorganischen Material ab. Das Filtrat wird im Vakuum vom Lösungsmittel befreit. Man erhält so 6,0 g eines Produktes mit einem Gehalt von 87% von 4-Fluor-3-phenoxy-benzaldehyd-ethylenacetal (Entspricht einer Ausbeute von 80% der Theorie).

Beispiel 2

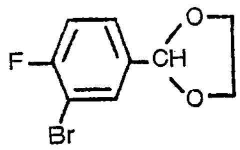

Eine Mischung aus 20,3 g (0,1 Mol) 3-Brom-4-fluor-benzaldehyd und 6,8 g (0,11 Mol) Ethandiol-1,2 wird mit 26 g (0,24 Mol) Trimethylchlorsilan versetzt und 3 Stunden auf 100°C erhitzt. Nach Abkühlen auf Raumtemperatur gibt man 100 ml Toluol zu und schüttelt 2 mal mit je 50 ml Eiswasser. Die organische Phase wird über Natriumsulfat getrocknet und im Vakuum eingedampft. Den Rückstand destilliert man im Vakuum. Man erhält so 21 g (85% der Theorie) 3-Brom-4-fluor-benzaldehydethylenacetal in Form eines farblosen Öles mit dem Siedepunkt 79—81°C/0,1 mm Hg.

Beispiel 3

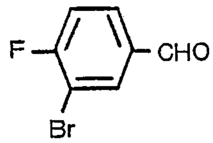 (III)

Variante (a)

Eine Lösung von 45,9 g (0,225 Mol) 3-Brom-4-fluor-benzylalkohol in 45 ml Methylenchlorid wird zu einer Suspension von

78 g 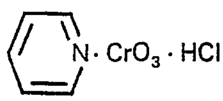

in 450 ml Methylenchlorid getropft. Dabei steigt die Temperatur des Gemisches bis etwa 40°C an. Man rührt anschließend 1 Stunde nach, dekantiert die organische Phase von den Chromsalzen und destilliert das Lösungsmittel im Vakuum ab. Der Rückstand wird destilliert. Man erhält so 39 g (86% der Theorie) 3-Brom-4-fluor-benzaldehyd mit dem Siedepunkt 63—65°C/0,3 mm Hg.

Variante (b)

Zu einer Mischung von 10 g Salpetersäure (Dichte 1,4) und 5 ml Wasser gibt man bei 30—35°C 10,2 g (0,05 Mol) 3-Brom-4-fluor-benzylalkohol und rührt das Gemisch anschließend 5 Stunden bei Raumtemperatur. Nach Zugabe von 30 g Eis versetzt man die Mischung mit Natronlauge, bis ein pH-Wert von 13 erreicht ist und schüttelt dann mit 200 ml Toluol aus. Die organische Phase wird drei mal mit je 50 ml Wasser gewaschen, über Natriumsulfat getrocknet und dann eingedampft. Der Rückstand wird destilliert. Man erhält so 6,1 g (60% der Theorie) 3-Brom-4-fluor-benzaldehyd in Form eines langsam erstarrenden Öles mit dem Schmelzpunkt 29—31°.

Variante (c)

Zu einer Lösung von 20,5 g (0,1 Mol) 3-Brom-4-fluor-benzylalkohol in 250 ml Methylenchlorid gibt man bei Raumtemperatur eine Mischung aus 29,4 g (0,3 Mol) Schwefelsäure, 50 ml Wasser und 2 ml Aliquat 336 (Tricaprylmethylammoniumchlorid). Danach versetzt man Reaktionsgemisch mit 9,7 g (0,033 Mol) Kaliumdichromat und hält die Temperatur während 2 Stunden durch schwaches Kühlen auf ca. 25°C. Nach Zugabe von 100 ml Wasser trennt man die organische Phase ab und schüttelt das Wasser noch einmal mit 100 ml Methylenchlorid aus. Die organischen Phasen werden 2 mal mit je 100 ml Wasser, dann einmal mit 100 ml gesättigter Natriumhydrogencarbonatlösung und noch einmal mit 100 ml Wasser gewaschen, getrocknet, über Natriumsulfat und im Vakuum eingedampft. Der Rückstand wird destilliert. Man erhält auf diese Weise 16,3 g (81% der Theorie) 3-Brom-4-fluor-benzaldehyd als farbloses Öl mit dem Siedepunkt 65°C/0,3 mm Hg.

Beispiel 4

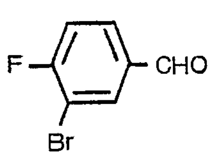 (III)

Zu einer Mischung aus 166 g Aluminiumchlorid und 150 ml 1,2-Dichlorethan werden bei einer Innentemperatur von 30°C 62 g 4-Fluor-benzaldehyd tropfenweise gegeben und das Gemisch wird etwa 30 Minuten nachgerührt. Dann werden 88 g Brom bei einer Innentemperatur zwischen 30 und 40°C zugetropft. Das Reaktionsgemisch wird etwa 2 Stunden nachgerührt und dann auf Eis gegossen. Nach Abtrennen der organischen Phase wird die wässrige Phase mit 1,2-Dichlorethan nachextrahiert. Die vereinigten organischen Phasen werden mit Wasser gewaschen, mit Natriumsulfat getrocknet und filtriert. Nach Abdestillieren des Lösungsmittels unter vermindertem Druck erhält man als Rückstand ein Rohprodukt, welches nach gaschromatographischer Analyse 78,8% 3-Brom-4-fluor-benzaldehyd und 17,6% 4-Fluor-benzaldehyd enthält. Nach Vakuumdestillation erhält man 58 g 3-Brom-4-fluor-benzaldehyd von Siedepunkt 108°C/25 mBar und vom Brechungsindex $n_D^{20}$: 1,5737. Das Produkt erstarrt allmählich: Schmelzpunkt 30—31°C.

Beispiel zur Herstellung von 4-Fluor-3-phenoxy-benzaldehyd:

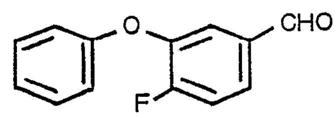

Eine Lösung von 26 g (0,1 Mol) 4-Fluor-3-phenoxy-benzaldehyd-ethylenacetal in 60 ml Ethanol, 20 ml Wasser und 1 ml konzentrierter Salzsäure wird 3 Stunden bei Raumtemperatur aufbewahrt. Dann destilliert man das Ethanol im Vakuum ab und versetzt den Rückstand mit 100 ml Toluol. Das Wasser wird abgetrennt, die organische Phase zwei mal mit je 50 ml Wasser gewaschen, über Natriumsulfat getrocknet und im Vakuum eingedampft. Den Rückstand destilliert man im Vakuum. Man erhält auf diese Weise 19,6 g (91% der Theorie) 4-Fluor-3-phenoxy-benzaldehyd in Form eines farblosen Öles mit dem Siedepunkt 102—104°C/0,1 mm Hg.

**Patentansprüche**

1. 4-Fluor-3-phenoxy-benzaldehyd-acetale der Formel I

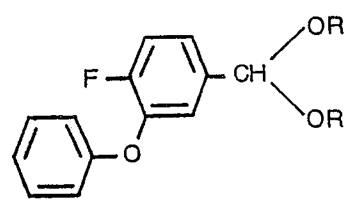

(I)

in welcher die beiden Reste

R einzeln für $C_{1-4}$-Alkyl oder zusammen für $C_{2-5}$-Alkandiyl stehen.

2. Verfahren zur Herstellung der Verbindungen der Formel (I),

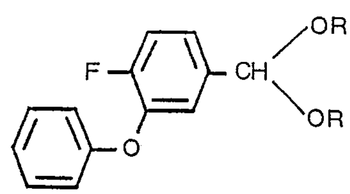

(I)

in welcher die beiden Reste

R einzeln für $C_{1-4}$-Alkyl oder zusammen für $C_{2-5}$-Alkandiyl stehen,

dadurch gekennzeichnet, daß man 3-Brom-4-fluor-benzaldehyd-acetale der Formel II

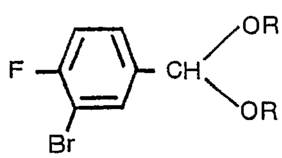

(II)

in welcher

R die oben angegebene Bedeutung hat,

mit Alkali- oder Erdalkali-phenolaten in Gegenwart von Kupfer oder Kupferverbindungen als Katalysatoren und in Gegenwart eines aprotisch polaren Verdünnungsmittels sowie gegebenenfalls in Gegenwart von Hilfsstoffen aus der Reihe der Alkali- oder Erdalkali-halogenide oder -carbonate und gegebenenfalls in Gegenwart eines basischen Entwässerungsmittels bei Temperaturen zwischen 100 und 200°C umsetzt.

3. Verwendung der Verbindungen der Formel (I) als Zwischenprodukte zur Herstellung von 4-Fluor-3-phenoxy-benzaldehyd durch Umsetzung mit Säuren nach an sich bekannten Acetalspaltungsmethoden.

4. 3-Brom-4-fluor-benzaldehyd sowie seine Acetale der Formel III und II

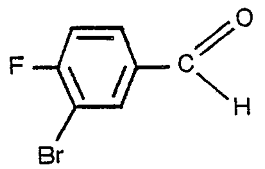

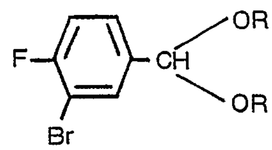

III II

welcher die beiden Reste einzeln für $C_{1-4}$-Alkyl oder zusammen für 2—5-Alkandiyl (Alkylen) stehen.

**Revendications**

1. 4-Fluoro-3-phénoxybenzaldéhyde-acétals de formule I

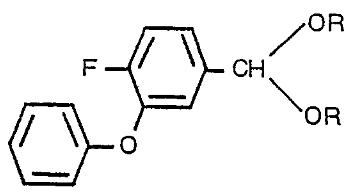

(I)

dans laquelle les deux restes

R représentent individuellement un groupe alkyle en $C_1$ à $C_4$ ou forment ensemble un groupe alcanediyle en $C_2$ à $C_5$.

2. Procédé de production des composés de formule (I)

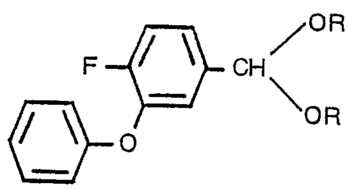

(I)

dans laquelle les deux restes

R représentent individuellement un groupe alkyle en $C_1$ à $C_4$ ou forment ensemble un groupe alcanediyle en $C_2$ à $C_5$,

caractérisé en ce qu'on fait réagir des 3-bromo-4-fluorobenzaldéhyde-acétals de formule II

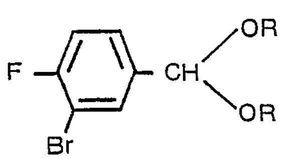

(II)

dans laquelle

R a la définition indiquée ci-dessus,

avec des phénolates alcalins ou alcalino-terreux en présence de cuivre ou de composés de cuivre comme catalyseurs et en présence d'un diluant aprotique polaire ainsi que, le cas échéant, en présence d'adjuvants de la série des halogénures ou des carbonates de métaux alcalins ou alcalino-terreux, et en la présence éventuelle d'un agent déshydratant basique, à des températures comprises entre 100 et 200°C.

3. Utilisation des composés de formule (I) comme produits intermédiaires pour l'obtention de 4-fluoro-3-phénoxybenzaldéhyde par réaction avec des acides par des procédés connus de décomposition d'acétals.

4. 3-Bromo-4-fluorobenzaldéhyde ainsi que ses acétals de formules III et II

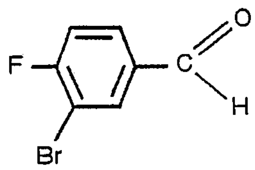

III

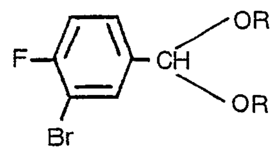

II

dans lesquelles les deux restes

R représentent individuellement un groupe alkyle en $C_1$ à $C_4$ ou forment ensemble un groupe alcanediyle (alkylène) en $C_2$ à $C_5$.

## Claims

1. 4-Fluoro-3-phenoxy-benzaldehyde acetals of the formula I

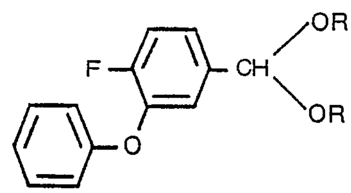

(I)

in which

the two radicals R individually represent $C_{1-4}$-alkyl or together represent $C_{2-5}$-alkanediyl.

2. Process for the preparation of the compounds of the formula (I),

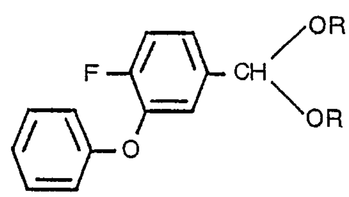

(I)

in which

the two radicals R individually represent $C_{1-4}$-alkyl or together represent $C_{2-5}$-alkanediyl, characterised in that 3-bromo-4-fluoro-benzaldehyde acetals of the formula II

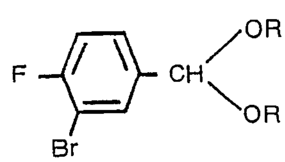

(II)

in which

R has the meaning indicated above,

are reacted with alkali metal phenolates or alkaline earth metal phenolates in the presence of copper or copper compounds as catalysts and in the presence of an aprotic polar diluent and if appropriate in the presence of auxiliaries from the series comprising alkali metal halides and carbonates and alkaline earth metal halides and carbonates, and if appropriate in the presence of a basic dehydrating agent, at temperatures between 100 and 200°C.

3. Use of the compounds of the formula (I) as intermediates for the preparation of 4-fluoro-3-phenoxybenzaldehyde by reaction with acids by known methods for splitting acetals.

4. 3-Bromo-4-fluoro-benzaldehyde and acetals thereof, of the formula III and II

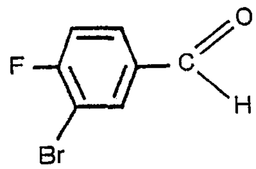

III

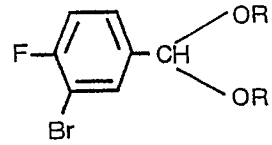

II

in which

the two radicals R individually represent $C_{1-4}$-alkyl or together represent 2—5-alkanediyl (alkylene).